# EUROPEAN PATENT SPECIFICATION

(11) **EP 4 370 145 B1**
(45) Date of publication and mention of the grant of the patent: **26.08.2026**
(21) Application number: 21743200.4
(22) Date of filing: 16.07.2021
(51) Int. Cl.: A61K 38/10, A61K 36/22, A61P 37/08

(54) **IMMUNOTHERAPEUTIC METHOD FOR INCREASING CASHEW TOLERANCE**
IMMUNTHERAPIE ZUR ERHÖHUNG DER TOLERANZ VON CASHEW-NÜSSEN
PROCÉDÉ IMMUNOTHÉRAPEUTIQUE PERMETTANT D'AUGMENTER LA TOLÉRANCE AUX NOIX DE CAJOU

(43) Date of publication of application: 22.05.2024
(73) Proprietor: DBV Technologies, 92320 Chatillon (FR)
(72) Inventor: DIOSZEGHY, Vincent, 92120 Montrouge (FR); MARTIN, William, PROVIDENCE, RI 02909 (US); DE GROOT, Anne, PROVIDENCE, RI 02909 (US)
(74) Representative: Cabinet Becker et Associés
(86) International application number: PCT/EP2021/070036
(87) International publication number: WO 2023/284985

(56) References cited:
- EP-A1- 2 217 272
- WO-A1-2019/010471
- WO-A2-2004/042026
- US-A1- 2003 124 060
- US-A1- 2019 231 892
- US-A1- 2020 010 530
- US-B2- 7 884 184
- BENJAMIN PELLETIER ET AL: "Epicutaneous immunotherapy protects cashew-sensitized mice from anaphylaxis", ALLERGY, WILEY-BLACKWELL PUBLISHING LTD, UNITED KINGDOM, vol. 76, no. 4, 23 October 2020 (2020-10-23), pages 1213 - 1222, XP071463481, ISSN: 0105-4538, DOI: 10.1111/ALL.14605

## Description

### FIELD OF THE INVENTION

The present invention relates to an immunotherapeutic method for increasing tolerance to cashew in an allergic subject.

### BACKGROUND OF THE INVENTION

The cashew plant (Anacardium occidentale) is a tropical evergreen tree belonging to the *Anacardiaceae* family. It produces cashew seeds (nuts) that are regularly consumed by most of the world's population.

Unfortunately, cashew is also classified as one of the most potent allergenic food, and no approved specific treatment is available to address this issue. The prevalence of cashew allergy has risen over the last decades in industrial countries with the increasing consumption of this nut. Besides, cashew allergy was shown to cause a higher rate of severe reactions than peanut allergy, including wheezing, cardiovascular symptoms, severe dyspnoea and collapse (Clark, Allergy, 2007:62-913-916). The mere exposure to cashew by smelling, touching or tasting (without eating) was reported to be sufficient to trigger an allergic reaction in cashew-allergic patients.

In spite of its increasing prevalence, there is no available, authorized, medical treatment enabling to manage cashew allergy. This lack of therapeutic treatments can be explained by the unique severity of anaphylactic reactions triggered by the consumption of cashew-containing food in allergic individuals. As of today, the sole option offered to allergic patients is strict avoidance of cashew in diet and emergency treatments in case of allergic treatments such as self-injectable epinephrine or intravenous antihistamine. But such an avoidance is not straightforward: cashew nuts are often packaged with other nut types. Besides, cashew nuts can be hidden in a wide variety of commonly ingested foods, such as Asian meals, sweets, ice creams, cakes, chocolates and sauces.

Pelletier, 2021 (Allergy, 2021;76:1213-1222) relates to the assessment of epicutaneous immunotherapy in an animal model of cashew allergy.

International application WO2009/071599 relates to an immunotherapeutic method for increasing groundnut tolerance in a subject by epicutaneous route by means of a skin patch.

US patent 7,884,184 relates to T cell epitopes derived from immunoglobulin and their medical uses.

As of today, there is thus a need of immunotherapeutic treatment enabling to increase tolerance to cashew allergen and thus avoid severe reaction in subjects allergic to cashew nut.

### SUMMARY OF THE INVENTION

The invention is as defined in the appended set of claims.

In a first aspect, the invention relates to a cashew allergen for use in increasing tolerance to cashew in a subject, wherein the cashew allergen is administered in combination with at least one regulatory Treg peptide by epicutaneous route and wherein:
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof, and
- the at least one regulatory Treg peptide is able to cause a tolerogenic response and consists of:
   - a polypeptide consisting of SEQ ID NO:2 or SEQ ID NO:3,
   - a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
   - a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications.

The cashew allergen may be a protein extract from cashew nut, preferably obtained from defatted cashew nut flour.

In a preferred embodiment, the cashew allergen and the at least one regulatory Treg peptide are simultaneously applied at the same skin area in the subject.

Typically, the cashew allergen and the at least one regulatory Treg peptide are applied on intact skin, preferably by means of a skin patch.

The skin patch can be of any type. Preferably, the skin patch comprises a backing having a periphery adapted to create a hermetically closed chamber when applied on the subject's skin. The cashew allergen and the at least one regulatory Treg peptide are present in dry form, optionally in admixture with one or several pharmaceutically acceptable excipients, on the backing of the patch facing the skin. When applied on the skin, the cashew allergen and the at least one regulatory Treg peptide absorbed on the backing are solubilized by trans-epidermal water loss whereby they can cross the *stratum corneum* and penetrate intro epidermis so as to reach epidermal dendritic cells. Typically, the patch comprises a backing preferably made of polyethylene terephthalate (PET) and an adhesive foam crown, the adhesive foam crown being adapted to form an airtight joint between the backing of the patch and the skin of the subject whereby a hermetically closed chamber is obtained when the patch is applied on the skin.

In some embodiments, the cashew allergen and the at least one regulatory Treg epitope are simultaneously applied on the skin every day or every two days by means of a skin patch during at least 6 months, preferably at least 12 months.

The repeated administration of cashew allergen in combination of the at least one regulatory Treg epitope by epicutaneous route may result in a decrease in cashew-specific IgE plasma level and an increase is in cashew-specific IgG4 plasma level in the subject. Furthermore it may enable to establish more rapidly an increase in cashew tolerance in the subject as compared to the repeated administration of cashew allergen without the at least one regulatory Treg epitope. The invention may protect the subject from developing severe allergic reactions such as anaphylaxis and/or mast cell degranulation following accidental exposure to cashew, in particular by oral route.

The subject can have been diagnosed as allergic to cashew or be at risk of being allergic to cashew. Typically, the cashew allergen is is for providing desensitization in a subject allergic to cashew.

The invention also relates to regulatory Treg peptide derived from human IgG for use in increasing the responsiveness of a subject to desensitization with cashew allergen, wherein:
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof,
- the at least one regulatory Treg peptide is able to cause a tolerogenic response and consists of:
   - a polypeptide consisting of SEQ ID NO:2 or SEQ ID NO:3,
   - a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
   - a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications, and
- the regulatory Treg peptide and the cashew allergen are administered by epicutaneous route, preferably by means of a skin patch.

At last, the invention also relates to a skin patch comprising a backing having a periphery adapted to create a hermetically closed chamber when applied on the subject's skin, wherein
- the skin patch comprises a cashew allergen and at least one regulatory Treg peptide present in dry form, optionally in admixture with one or several pharmaceutically acceptable excipients, on the skin facing surface of the patch backing,
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof, and
- the at least one regulatory Treg peptide is able to cause a tolerogenic response and consists of:
   - a polypeptide consisting of SEQ ID NO:2 or SEQ ID NO:3,
   - a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
   - a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications.

### LEGEND TO THE FIGURES

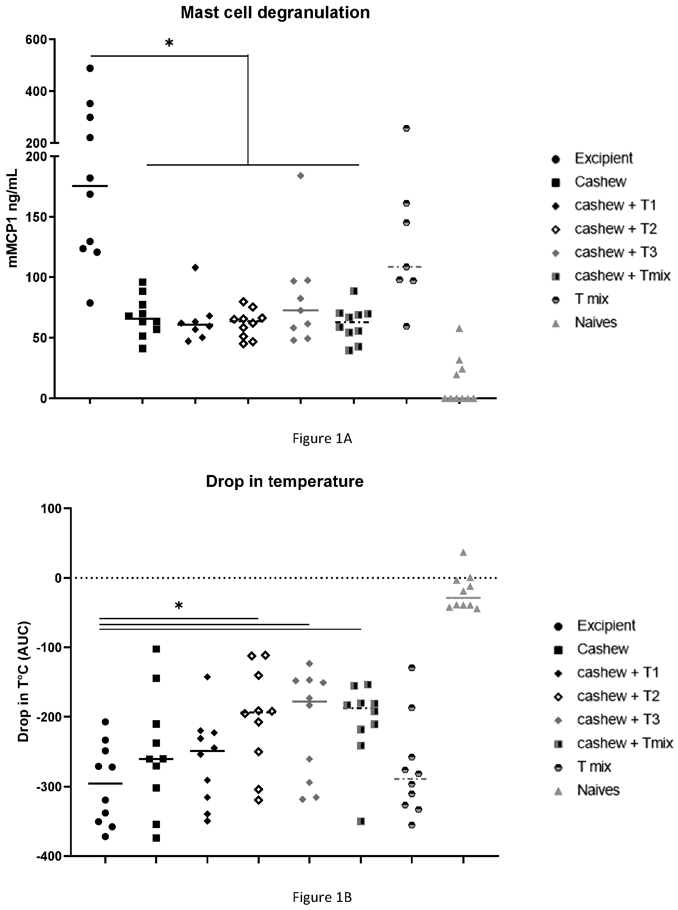

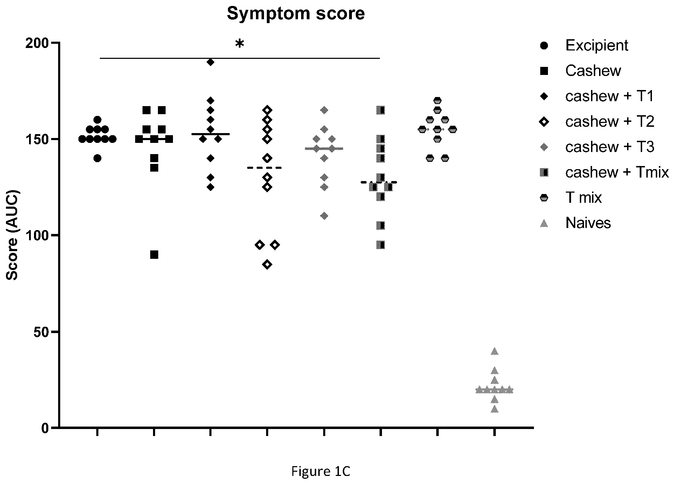

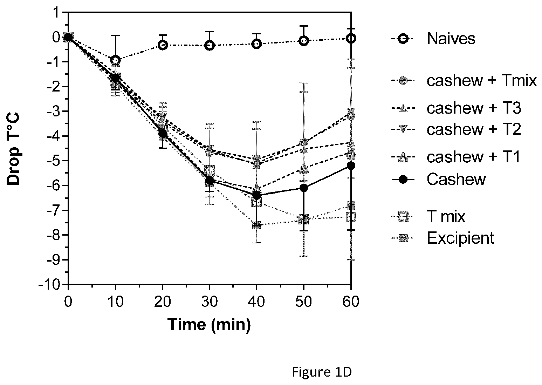

**Figure 1****: Evaluation of the level of protection afforded by EPIT against anaphylaxis following 8 weeks of treatment.** Mice were orally sensitized to cashew and treated by EPIT with VIASKIN containing either excipient, cashew, cashew with different murine Tregitopes or mix of Tregitopes alone. Following 8 weeks of EPIT, mice were challenged orally to cashew. (A) Evaluation of mast-cell activation: Blood samples were collected 60 minutes after the challenge to isolate plasma. mMCP-1 concentrations were measured from plasma by ELISA. (B, D and E) Body temperature was measured every 10 minutes following challenge for 60 minutes. The area under the curve (AUC) was calculated for each mice and showed with median of each group (B) Median and interquartile ranges of individual values of the kinetic of temperature drop is showed. (D). (C) Clinical symptoms were monitored every 10 minutes following challenge for 60 minutes. Data are Mean with SEM of individual values. P values were determined according to the Mann-Whitney test.

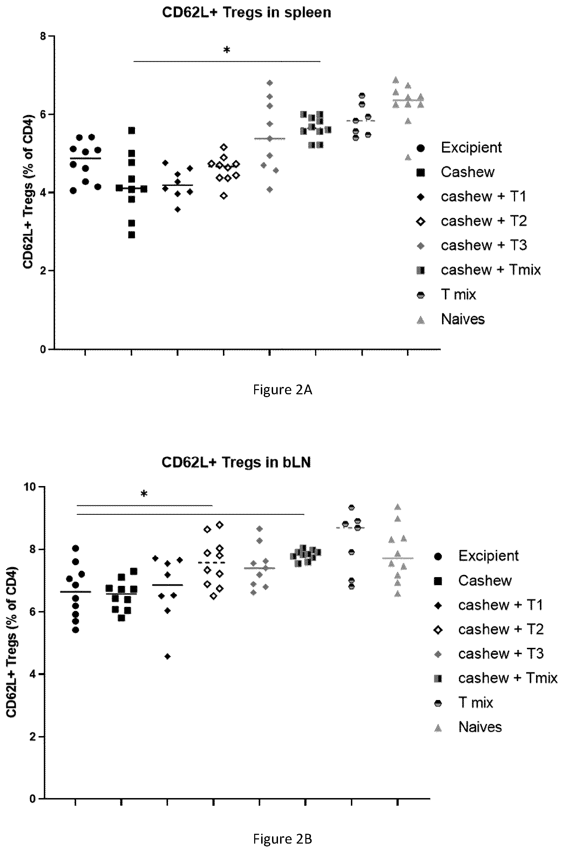

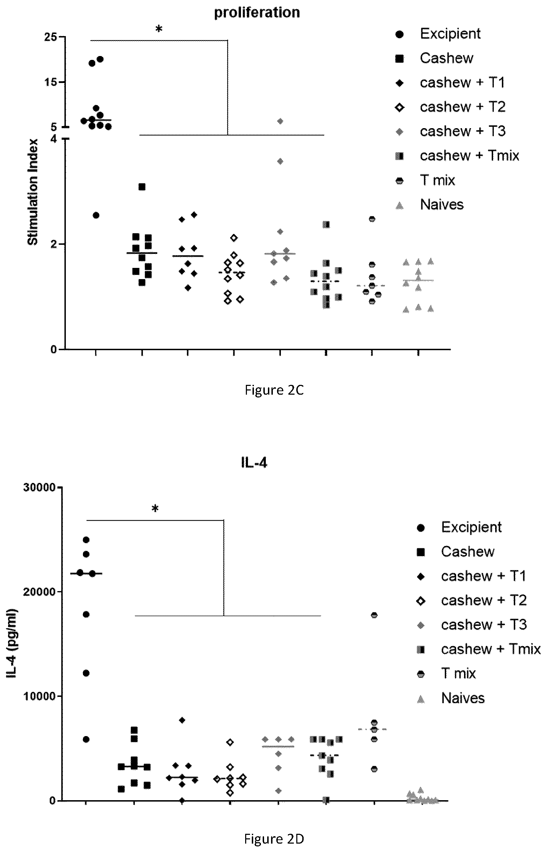

**Figure 2****: Cellular response induced by EPIT after 8 weeks of treatment.** Mice were orally sensitized to cashew and treated by EPIT with VIASKIN containing either excipient, cashew, cashew with different murine Tregitopes or mix of Tregitopes alone. At the end of experiment, animals were sacrificed to collect spleen and brachial lymph nodes. Cells from spleen (A) and LN (B) were isolated for phenotyping of Tregs by FACS (example of staining: LiveDead, CD3, CD4, CD25, FoxP3, LAP, CD62L). Cells from spleen were also used for in vitro culture with or without cashew stimulation for proliferation and cytokine production analysis (C and D).

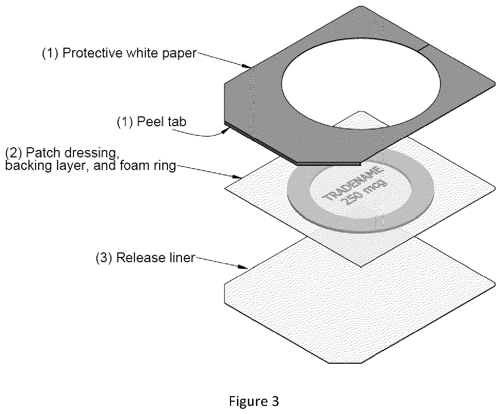

**Figure 3** shows an example of patch which can be used to perform the invention. Proceeding from the outer surface towards the surface adhering to the skin, the layers are: a peel-off protective paper with a peel tab (1); an adhesive dressing which ensures the adhesiveness of the patch on the skin and which covering a rounded backing layer, the rounded backing layer, smaller than the adhesive dressing containing the active substance (herein the regulatory Treg epitope and the cashew allergen), and an adhesive foam ring surrounding the active substance and fixed on the backing (2). The adhesive dressing is covered by a removable, release liner (3). For application on the skin, the release liner is removed, the patch is applied on the skin and the paper applicator is removed. The adhesive foam and the backing form together with the skin surface a hermetic condensation chamber enabling perspiration to occur and thus solubilization of the active substance present on the backing's surface facing the skin.

### DETAILED DESCRIPTION OF THE INVENTION

The references to methods of treatment in this description are to be interpreted as references to the compounds (e.g. the cashew allergen, the Regulatory Treg peptide or combination thereof as described herein), pharmaceutical compositions and medicaments of the present invention for use in a method for treating a subject by therapy, e.g. for increasing tolerance to cashew in a subject.

### General Definitions

As used therein, *"epicutaneous administration or application"* designates the application of a compound on a surface of the skin of a subject under conditions allowing a contact with the surface of the skin. Epicutaneous administration typically comprises skin application under condition sufficient to allow penetration or diffusion of the compound in the superficial layer(s) of the skin, preferably in the epidermis layers so that the compound can reach the epidermal dendritic cells such as Langerhans cells. Epicutaneous administration is preferably performed on intact and/or healthy skin area.

As used herein, *"Epidermis"* refers to the outlayer of the skin, the inner layers being the dermis and hypodermis. The epidermis is composed of 4 or 5 layers depending on the region of the skin being considered. These layers, in descending order, are the *stratum corneum,* the *stratum lucidum,* the *stratum granulosum,* the *stratum spinosum* and the *stratum basale.*

The *stratum corneum* is mostly composed of corneocytes which correspond to keratinocytes in their last stage of differentiation. The *stratum corneum* has a thickness generally ranging from 10 to 40 µm depending on the location of the skin and the age of the subject.

As used herein, *"an intact skin area"* refers to a skin area which has not been pretreated before the application of the compound(s) of interest (herein the cashew allergen and the regulatory Treg peptide)

As described herein, *"immunotherapy or immunotherapeutic treatment"* refers to a treatment wherein a desired immune reaction is induced in a patient in order to improve his/her condition. Immunotherapeutic treatment encompasses the treatment and/or the prevention of a disorder in the subject. As used herein, a disorder encompasses any allergy, any disorder, symptom or reaction caused or associated with an allergy.

As described herein *"epicutaneous immunotherapy"* or "EPIT" refers to an immunotherapy treatment wherein compound(s) of interest able to induce a desired immune reaction is administered by epicutaneous route, as defined above, e.g. by means of a skin patch.

As described herein, the term *"desensitization"* or *"desensitizing"* typically designates the sequential administration of one or more allergens to an allergic subject, to induce or increase a tolerance to said allergen(s) in said allergic subject.

The term "tolerance" is here defined as a reduction in immunological reactivity of a subject towards specific allergens. An "Increase or improvement in tolerance" can be evidenced by any means known by the skilled artisan, in particular by performing an oral challenge. When oral challenge is used, an "increase in tolerance" is observed when the oral dose of allergen necessary to trigger allergic reaction in the subject after the treatment (herein EPIT) is higher than that before the treatment. Typically said increase in dose may be of at least 1.1-fold, such as at least 1.5, 2.0, 3.0, 5.0, 10 or 100-fold.

As used herein, a *"compound"* designates any molecule able to induce a desired immune response in a subject in need thereof, such as a cashew allergen and/or a regulatory Treg peptide.

The terms *"polypeptide", "peptide"* and *"protein"* are used interchangeably herein to refer to a polymer of amino acid residues. The terms also apply to amino acid polymers in which one or more amino acid residues may be modified or non-naturally occurring residues, such as an artificial chemical mimetic of a corresponding naturally occurring amino acid. It should be understood that the term "protein" also includes fragments or variants of different allergens, such as epitope-containing fragments,.

As used herein, by "amino acid modification" is meant a change in the amino acid sequence of a polypeptide. "Amino acid modifications" which may be also termed "amino acid changes", herein include amino acid mutations such as substitution, insertion, and/or deletion in a polypeptide sequence. By "amino acid substitution" or "substitution" herein is meant the replacement of an amino acid at a particular position in a parent polypeptide sequence with another amino acid. By "amino acid insertion" or "insertion" is meant the addition of an amino acid at a particular position in a parent polypeptide sequence. By "amino acid deletion" or "deletion" is meant the removal of an amino acid at a particular position in a parent polypeptide sequence. The amino acid substitutions may be conservative. A conservative substitution is the replacement of a given amino acid residue by another residue having a side chain ("R-group") with similar chemical properties (e.g., charge, bulk and/or hydrophobicity). In general, a conservative amino acid substitution will not substantially change the functional properties of a protein or peptide. Conservative substitutions and the corresponding rules are well-described in the state of the art.

The term *"allergen"* as used therein, designates any molecule, substance or mixture of molecules which can produce an immune response resulting in an allergic reaction in a subject. Allergens may be of various nature, form and origin. These may be proteins (or polypeptides or peptides), lipids, sugars, etc., which may be in the form of extraction products, in a recombinant form, and/or of synthetic origin. The allergens may be in a native form, or in a fragmented form, denaturated form, etc.

In the context of the invention, the term "allergen" refers to allergenic molecule(s) derived from cashew nuts, in particular to allergenic proteins and isoforms derived from cashew nuts as well as variants and fragments thereof.

The allergen used to implement the method of the invention may comprise several allergenic molecules, in particular several allergenic proteins or peptides. The allergen may be typically in the form of an extract obtained from cashew nuts.

The allergen may be formulated with any excipient or carrier adapted to pharmaceutical use. The resulting allergenic preparation may be in different forms, such as for example as a liquid or solid. In a preferred embodiment, the allergen is in dry form, i.e. in freeze-dried or electrosprayed form.

By *"immunoglobulin"* as used herein is meant the structure that constitutes the natural biological form of an antibody, including variable and constant regions. In most mammals, including human and mice, the structure of full-length antibodies is generally a tetramer. Said tetramer is composed of two identical pairs of polypeptide chains, each pair having one *"light"* chain (typically having a molecular weight of about 25 kDa) and one *"heavy"* chain (typically having a molecular weight of about 50-70 kDa). In the case of human immunoglobulins, light chains are classified as kappa and lambda. The kappa (κ) chain is encoded by the immunoglobulin kappa locus on chromosome 2 and the lambda (λ) chain is encoded by the immunoglobulin lambda locus on chromosome 22. The two light chains in a naturally-occurring antibody are identical. Each light chain is composed of one constant (CL) domain and one variable domain (VL) that is important for binding antigen. There are several allotypes for kappa light chain, namely Km1, Km1,2 and Km3. Heavy chains are classified as mu, delta, gamma, alpha, or epsilon, and define the antibody's isotype as IgM, IgD, IgG, IgA, and IgE, respectively. IgG has several subclasses, including, but not limited to IgG1, IgG2, IgG3, and IgG4.

### - The Invention

Desensitization is the only available treatment that can modify the natural course of allergic diseases, by reducing sensitivity to allergens. In this method, a dose of an allergen is repeatedly administered in order to progressively induce an immune response characterized by tolerance to the allergen. This method is particularly indicated for patients with severe allergic IgE-dependent reactions. Even though desensitization has been in practice for a long time, the exact mechanism of its action is still not clear. In humans, it involves (i) an increase of IgG, in particular IgG4 which is a blocking antibody that may block IgE mediated mechanisms by inhibiting the release of inflammatory mediators from mast cells and basophils, (ii) an increase of regulatory T cells (Treg) leading to a better balance of the Th2 / Th1 profile, and (iii) the production of T cells producing IL-10 which counteracts the inflammatory effect of mast cells and promotes the production of IgG4. Desensitization is usually performed by sublingual (SLIT) or subcutaneous (SCIT) immunotherapy.

The Applicant investigated a new administration route of allergen to promote desensitization, namely the epicutaneous route. More precisely, the Applicant developed an immunotherapeutic treatment for the management of peanut allergy based on the repeated administration of peanut allergens by epicutaneous route by means of an in-house skin patch (called Viaskin). The marketing application for this treatment is under review before the European Medecine Agency (EMA) and the Food and Drug Administration (FDA).

Epicutaneous route enables to deliver the allergens to the epidermal dendritic cells such as Langerhans cells which process the allergens, migrate to lymph nodes and present epitopes to T-cells. Of note, the epicutaneous route does not result in the passage of the allergens into the bloodstream. The occurrence of severe systemic adverse reactions is very low with epicutaneous immunotherapy (EPIT) as compared to traditional desensitization protocols performed by sublingual or subcutaneous route.

The Applicant sought to assess epicutaneous immunotherapy (EPIT) in the management of cashew nut allergy. As illustrated in Pelletier, 2021 (Allergy, 2021;76:1213-1222), the Applicant developed a robust mouse model of IgE-mediated cashew-induced anaphylaxis.

The capacity of epicutaneous immunotherapy (EPIT) was then assessed in this model: cashew-sensitized mice were treated by repeated epicutaneous administrations of cashew allergens by mean of a skin patch up to 16 weeks and then undergo an oral challenge. The Applicant showed that EPIT was effective to protect against anaphylactic symptoms following oral challenge in sensitized mice treated with EPIT during 16 weeks. However, this protective effect was very low in mice treated during 8 weeks or 12 weeks whereas a very high protective effect against anaphylaxis was obtained in 8 weeks in a similar model of peanut-sensitized mice only. Furthermore, EPIT showed a more pronounced increase in Tregs in draining lymph nodes and spleen and a more pronounced decrease in allergen-specific IgE in peanut-sensitized mouse model than in cashew nut-sensitized model. These differences in terms of response to EPIT in peanut and cashew-sensitized models could result from the more immunogenic properties of cashew nuts. These results raised concerns about the applicability of EPIT in the management of cashew nut allergy: EPIT might be poorly accepted by patients if a limited increase in cashew tolerance is obtained after a very long treatment only.

Thus, the Applicant sought a strategy to increase the therapeutic effect of EPIT in the management of cashew nut allergy a.

In that context, the Applicant showed that the protective effect of EPIT can be potentiate by co-administering cashew allergens with regulatory T-epitopes derived from Immunoglobulin G (IgG) by epicutaneous route. The coadministration of cashew extract with regulatory T-reg epitopes accelerated the obtention of a protective effect : The decrease in cashew-specific IgE and increase in cashew-specific IgG2a after 8 weeks of treatment were more significant in cashew-sensitized mice co-administered with cashew extract and regulatory T-epitopes from IgG than in mice treated administered with the extract only. Similarly, the drop in temperature and the symptom score after oral challenge were significantly improved in mice cotreated with cashew extract and regulatory T-epitopes as compared to the other control groups after 8 weeks of treatment.

Of note, the increase in efficacy was particularly marked when the allergen extract was co-administered with mouse regulatory T-epitopes T2 and T3 which are autologous to hTreg-epitopes of SEQ ID NO:2 and NO:3 or mixtures thereof. In that case, an increase in CD62L+/FoxP3+ Treg induction were observed in skin draining lymph nodes as well as in spleen. This increase in Tregs was associated with reduced cashew specific T-cell proliferation and a down-modulation of Th2 cytokines (IL-4) in re-stimulated splenocytes.

In sum-up, the epicutaneous administration of regulatory Treg epitopes from IgG can significantly improve the efficacy of EPIT in the mouse model of IgE-mediated cashew-induced anaphylaxis.

Accordingly, the Invention relates to a method for increasing tolerance to cashew in a subject comprising co-administering the subject with a cashew allergen and at least one regulatory T-peptide by epicutaneous route.

More precisely, the invention relates to an immunotherapeutic method for increasing tolerance in a subject allergic to cashew wherein the subject is repeatedly co-administered with a cashew allergen and at least one regulatory T-peptide preferably derived from human immunoglobulin of G isotype by epicutaneous route.

The repeated combined administration of the cashew allergen and the least one regulatory T-peptide enables to progressively increase tolerance to cashew in the subject. Said increase of tolerance is higher and/or obtained more rapidly than in the case of repeated administration of the same cashew allergen by epicutaneous route but without that of the at least one regulatory Treg peptide.

In a particular aspect, the method of the invention is for providing desensitization to a subject allergic to cashew and/or is for treating cashew allergy. In an additional embodiment, the method of the invention is for decreasing the risk of onset, the severity and/or the frequency of allergic reactions, in particular severe reactions such as respiratory reactions, collapse and anaphylaxis in a subject allergic to cashew. Allergic reactions caused by cashew allergy are described further below.

In another aspect, the invention relates to a method for providing protection against anaphylaxis in case of accidental exposure to cashew in a subject allergic to cashew, which comprises repeatedly administering a cashew allergen in combination with at least one regulatory Treg epitope by epicutaneous route in the subject, wherein said repeated combined administration enables to progressively increase tolerance to cashew in the subject and wherein the increase of tolerance is higher and/or obtained more rapidly by the coadministration than the repeated administration of the same cashew allergen by epicutaneous route but without that of the regulatory Treg peptide.

In an additional aspect, the invention relates to a method for increasing the responsiveness of a subject allergic to cashew to desensitization with a cashew allergen, wherein the cashew allergen is repeatedly administered in combination with at least one regulatory Treg peptide by epicutaneous route.

In another aspect, the invention related to a method to potentiate epicutaneous immunotherapy (EPIT) in a subject allergic to cashew, wherein the subject is repeatedly administered with a cashew allergen in combination with at least one regulatory Treg peptide by epicutaneous route, said combined administration enabling to potentiate the decrease in cashew-specific IgE and/or the increase cashew-specific IgG4 and/or the induction of Treg cells, in particular CD4+/CD25+/FoxP3+ as compared to a similar EPIT wherein cashew allergen is administered without the at least one regulatory Treg peptide.

In another aspect, the invention relates to a cashew allergen for use in immunotherapy of a subject allergic to cashew, in particular for increasing tolerance to cashew in said subject, wherein the cashew allergen is repeatedly administered in combination with a regulatory Treg-peptide by epicutaneous route.

In a further aspect, the invention relates to a regulatory Treg peptide for use in increasing responsiveness of a allergic subject to desensitization, wherein the regulatory Treg peptide is administered in combination with a cashew allergen by epicutaneous route.

As fully described below, the cashew allergen and the regulatory T peptide can be administered simultaneously, separately (i.e. spread out over time) or consecutively on the same skin area or at different skin areas in the subject. In a preferred embodiment, the cashew allergen and the regulatory T peptide are administered simultaneously at the same skin area, e.g. by means of a skin patch. The regulatory T peptide and the cashew allergen are typically applied on an intact area of the skin and without any adjuvant.

It is described the use of a cashew allergen and a regulatory Treg peptide for the preparation of a pharmaceutical composition for increasing tolerance to cashew in a subject in need thereof, wherein the pharmaceutical composition is repeated administered by epicutaneous route. As fully described below, the pharmaceutical composition is preferably in dry form and delivered by means of a skin patch. The pharmaceutical composition may comprise one or several pharmaceutically acceptable excipients.

Another object of the invention also refers to a skin patch comprising both the cashew allergen and the regulatory T peptide, said skin patch being adapted to provide the administration of said compounds by epicutaneous route. As described further below, in certain embodiments, the skin patch device comprises a backing, the periphery of said backing being adapted to create with the skin a hermetically closed chamber, wherein the backing bears on its skin facing side within the chamber the cashew allergen and the regulatory Treg in dry form optionally in admixture with one or several pharmaceutically acceptable excipient.

### - The subject

The subject refers to any human being in need of immunotherapy with cashew allergen. Typically, the subject of interest has been diagnosed as being allergic to cashew. In certain embodiment, the subject is at risk of having or developing an allergy to cashew in particular due to a family history of cashew allergy and/or due to a previously diagnosed allergy, in particular a food allergy (e.g. peanut or pistachio) and/or to the presence of disorders underlying allergic pattern such as atopic dermatitis, allergic rhinitis or asthma.

Preferably, the subject has been diagnosed as being allergy to cashew. In other words, the subject is allergic to cashew.

The subject allergic to cashew can experiment a large variety of reactions upon exposure to cashew, in particular upon accidental ingestion of cashew.

Said allergic reactions encompass cutaneous, respiratory, cardiovascular and gastrointestinal reactions. These allergic reactions can develop into anaphylaxis, anaphylaxis having a significant incidence in cashew allergy.

Cutaneous manifestations seem to be the most frequent and encompass eye angioederma, lip angioedema, urticaria and perioral rash.

Gastrointestinal reactions encompass vomiting, diarrhea and abdominal pain.

Respiratory reactions encompass cough, shortness of breath, wheeze and sneezing.

The subject is a human. The patient may be of any gender and of any age. The subject may be a newborn, an infant, a child, a teenager, or an adult. Preferably, the subject is under 18 years old, preferably under 14 years old. For example, the subject is a child from 1 year old to 11 years old such as from 4 to 11 years old.

### - The cashew allergen

Cashew nuts refer to the seeds of a tropical evergreen, tree, *Anacardium occidentale,* also called cashew tree.

As used in the present specification, the term *"cashew allergen"* refers to any substance, molecule or mixture of molecules derived from cashew nuts which can produce an allergic reaction in a subject upon ingestion.

The term *"cashew allergen"* both refers to an isolated allergenic molecule but also to the combination of several allergenic molecules, e.g. as in the case of cashew extract or semipurified protein preparation obtained from cashew nuts. Said allergenic molecules are typically capable of causing an IgE-mediated systemic allergic reaction.

It is described that the cashew allergen comprises or essentially consists of an allergenic protein from cashew nut, an isoform, a variant, a fragment and a combination thereof.

As explained above, a variant or a fragment of an allergenic protein of interest comprises at least one IgE-binding epitope originally present in the allergenic protein.

Said allergenic proteins or polypeptides can be isolated by extraction process from cashew nuts, in particular from cashew kernels. They can be also recombinantly prepared or chemically synthesized. In some embodiments, said allergenic proteins can be in native form. In other embodiments, said allergenic proteins can be denatured or chemically modified.

Cashew nuts is mainly composed of lipids (44% by weight) and proteins (19% by weight).

The three main allergenic proteins present in cashew nuts are :
- Ana o 1, 7S vicilin seed storage protein of 50kDa that is expected to form a trimer in the native state and which was characterized in Wang, J allergy clin Immunol, V110, pages 160-166 (doi:10.1067/mai.2002.125208)
- Ana o 2, a 11S globulin which consists of a 33kDa large subunit and a 20kDa small subunit, bound together by disulfide bonds, forming a 53kDa protein. Ana o 2 is also referred to anacardein. For further information see Wang, 2003, Int Arch Allergy Immunol, 132:27-39
- Ana o 3, a 2S albumin of 12.6kDa. For further information see Robertham, J allergy clin Immunol, 2005, V115, pages 1284-1290.

Isoforms of these proteins are disclosed in the art and are included in the instant invention. For instance isoforms of Ana o 1 encompass Ana o 1.0101 and Ana o 1.0102.

The amino acid sequences of Ana o 1, Ana o 2, and Ana o 3 (including isoforms) are known to the skilled person. As an illustration, the UniprotB accession numbers of Ana o 1, Ana o 2, Ana o 3 and some of their isoforms are for instance : Q8L5L5 and Q8L5L6, Q8GZP6, Q8H2B8 respectively.

These proteins can be purified from cashew nuts (see e.g. Reitsma, J. Agric. Food. Chem. 2016, 64, 5, 1191-1201) or recombinantly produced by a recombinant organism, such as genetically modified bacteria, e.g. E coli, yeasts or by any other methods known by the man skilled in the art.

These proteins can be used in combination or separately.

It should be understood that the term "protein derived from cashew" also includes fragments or variants of the above allergenic proteins, such as epitope-containing fragments, recombinantly proteins optionally having one or several amino acid modification and proteins obtained from cashew nuts which can optionally be subjected to enzymatic, chemical, mechanical or thermal treatment.

In the context of the invention, the cashew allergen comprises, or essentially consists of, an allergenic protein selected from the group consisting of Ana o 1, Ana o 2, Ana o 3, isoforms, fragments, and combinations thereof.

Preferred fragments of Ana o 1 encompass allergenic fragments comprising at least one IgE-binding epitope as shown in Table III in doi:10.1067/mai.2002.125208

Preferred fragments of Ana o 2 encompass allergenic fragments comprising at least one IgE-binding epitope as shown in Table 6 in Wang, 2003, Int Arch Allergy Immunol, 132:27-39

Preferred fragments of Ana o 3 encompass allergenic fragments comprising at least one IgE-binding epitope as shown in Table II, page 1289 of Robertham, J allergy clin Immunol, 2005, V115, pages 1284-1290.

In some embodiments, the cashew allergen is in the form of a composition comprising Ana o 1, Ana o 2, and Ana o 3, optionally in combination or in admixture with other molecules from cashew such as other proteins, polysaccharides and/or lipids from cashew.

In a particular embodiment, the cashew allergen is in the form of a cashew extract enriched in at least one protein selected from Ana o 1, Ana o 2 and Ana o 3.

A cashew extract designates any preparation (lysate, filtrate, homogenate, powder including defatted powder...) obtained from cashew nuts or cashew kernels. The cashew extract can be used directly. Alternatively cashew protein allergens can be at least partially isolated or purified from this extract, e.g. so as to obtained a protein extract or concentrate. Such a process can involve solvent extraction, filtration, centrifugation, precipitation, chromatography or any other techniques known by the skilled person.

In preferred embodiments, the cashew allergen is in the form of a protein extract or concentrate from cashew nuts. In other words, the cashew allergen can refer to partially isolated allergenic proteins. The protein extract from cashew nuts typically comprises at least one protein selected from Ana o 1, Ana o 2 and Ana o 3. Preferably, cashew allergen is in the form of a protein extract from cashew nuts comprising Ana o 1, Ana o 2 and Ana o 3.

Such a protein extract may have a total amount in proteins of at least 30%, preferably of at least 40%, 50%, 60%, 70%, 80%, 90% or 95% by weight as compared to the total weight of the extract.

The protein extract may be obtained by extracted defatted cashew flour with an appropriate solvent of interest.

For instance, the allergenic proteins can be obtained by extracting defatted cashew nut flour in PBS during an appropriate period of time (e.g. overnight under stirring and at 4°C). The solution is separated from insoluble residues by centrifugation and the protein extract/concentrate may be obtained from the solution e.g. by lyophilization. Alternative methods are also described in the prior art.

Besides, one can use commercially available cashew extracts as those provided by Stallergenes Greer.

The cashew allergen may be in any form such as in liquid form or in solid form such as in powder, lyophilizate and the like.

### - The regulatory Treg peptide

As used herein, a regulatory Treg peptide refers to a polypeptide comprising or consisting of a regulatory Treg epitope, a variant thereof, a retro-inverso analogue thereof, or a variant of retro-inverso analogue thereof.

A regulatory Treg peptide exhibits tolerogenic effects: in particular, a regulatory Treg peptide according to the invention is capable of inducing expansion of CD4+/CD25+/FoxP3+ T cells in vitro.

The capacity of a peptide to induce expansion of natural CD4+/CD25+/FoxP3+ T cells in vitro can be assessed by methods well known by the skilled artisan. For instance, one can proceed as follows: Human peripheral mononuclear cells (PBMCS) isolated from blood of human volunteers are stimulated directly ex vivo for 4 days in the presence of Tregitope alone, phytohemagglutinin alone (a mitogenic positive control) or no stimulus. 1 x 10⁶ cells were stained with anti-CD4-FITC ( clone RPA-T 4; eBioscience) and anti-CD25-5 APC ( clone BC96; eBioscience) antibodies for 30 minutes on ice in Flow Staining Buffer (eBioscience) and washed twice with buffer. Following cell surface staining, cells are fixed and permeabilized and stained intracellularly for FOXp3 (clone PCH101; eBioscience) following manufacturer's protocol. The frequency of FOXp3 positive CD4+/CD25+ T cells under various culture conditions is enumerated. T cell activation is indicated by increase in CD4+CD25+ expression, which, when accompanied by an increase in FOXp3 expression, is indicative that the activated cells are regulatory.

Alternatively, the validation of regulatory Treg peptides may be performed with an adapted Tetanus Toxoid Bystander Suppression Assay by measuring the inhibitory capacity of potential regulatory peptides on the recall response of human CD4 T cells to the tetanus toxoid (TT). Briefly, PBMCs are labeled with CFSE cell proliferation dye (eBioscience) and rest overnight at 37°C, 5% CO2. The following day, cells are stimulated with Tetanus toxoid (TT) (Astarte Biologics, cat no. 1002) at 0.5 mg/ml alone and in combination with the putative regulatory peptides or control peptide at 8, 16 or 24 mg/ml, then incubated for 6 days and analyzed by flow cytometry on day 7. CD4+ T cell proliferation, T effector activation and the ratio of regulatory to effector T cells are measured and reflect the tolerogen capacity of Treg peptide.

A regulatory Treg peptide has less than 50 amino acids in length, preferably less than 40 amino acids in length such as from 5 to 35 or from 7 to 30 amino acids in length.

The tolerogenic activities of these peptides derive from the Regulatory Treg epitope that they contain. Regulatory Treg epitope refers to linear sequences (generally of 15-30 amino acids in length) contained in common autologous proteins such as IgG and able to cause a tolerogenic response. Regulatory Treg epitopes can be found in conserved regions of IgG. Regulatory Treg epitopes are capable of binding to MHC molecules and engaging and/or activating circulating naturally occurring Tregs, leading to the expression of the immune suppressive cytokines including, but not limited to, IL-10 and TGF-b and TNF-a.

A variant of a regulatory Treg epitope refers to a polypeptide having an amino acid sequence which differs from the amino acid sequence of a regulatory Treg epitope in virtue of one or several amino acid modifications (e.g. 1, 2, 3, 4 and 5 modifications) while having tolerogenic activity, namely the capacity of inducing CD4+/CD25hi/FoxP3+ T cells in vitro.

A retro-inverso analogue of a parent peptide refers to a linear peptide whose amino acid sequence is reversed and the α-center chirality of the amino acid subunits is inverted as well as compared to the parent peptide. This type of peptide includes D-amino acids in the reverse sequence to help maintain side chain topology similar to that of the original L-amino acid parent peptide and make it more resistant to proteolytic degradation.

The first regulatory Treg epitopes (also called Tregitopes) were identified in the Fc and Fab regions of human IgG antibodies by Anne de Groot et coll. They showed that Tregitopes have the following four characteristics: (1) their sequences are highly conserved in similar autologous proteins; (2) they almost all comprise a single 9-mer frames predicted by their in house epitope prediction algorithm (EpiMatrix) to bind to at least four different HLA DR alleles and thus are likely to be broadly recognized in the human population; (3) in response to Tregitopes, T cells exhibit a T regulatory phenotype (CD4+CD25+FoxP3+); and (4) co-incubation of T cells with Tregitopes and immunogenic peptides inhibits effector T cell (Teff) response to the immunogenic peptides in vitro and suppresses antigen-specific secretion of effector cytokines responses (Cousens et al., Human Immunology, 2014, 75, 1139-1146). It was also shown that the IgG-derived Tregitopes are conserved across non-human species such as mouse, rat, cat, camel, cow and non-human primates.

Several Tregitopes derived from human IgG, in particular from the constant region of IgG e.g. the Fc region or in the Fab region e.g. in the kappa light chain of IgG are described in table 2 of WO2008094538. Said Tregitopes are described to bind MHC class II molecules, engage T cell receptor (TCR) in context of MHC class II molecules and activate natural regulatory T cells.

It is described a regulatory Treg peptide is derived from human IgG, which means that it comprises a Tregitope from a human IgG, in particular from Fc region or from the variable domain of Fab, such as in the framework of VL of kappa light chain, of a human IgG. In some embodiments, the Tregitope is selected from the tregitopes derived from human IgG showed in page 46, Table 2 of WO2008094538, variants thereof and retro-inverso analogues thereof. For instance, Tregitopes of interest are those identified as Tregitope-289, Tregitope-009, Tregitope-029 and Tregitope of SEQ ID NO:31 in page 46, Table 2 of WO2008094538.

Regulatory Treg peptides can be prepared by any methods known in the art, e.g. recombinantly or by chemical synthesis such as solid phase peptide synthesis.

Surprisingly, the Applicant showed that certain murine Tregitopes T2 and T3 which are autologous to human Tregitopes of SEQ ID NO:2 and 3 clearly potentiate the efficacy of EPIT for providing tolerance to cashew in the cashew-sensitized mouse model, while other Tregitopes have little effect (e.g. mouse Tregitope of SEQ ID NO:4 which is the homolog of human Tregitope of SEQ ID NO:1).

In the context of the invention, the regulatory Treg peptide consists of:
- a polypeptide having a sequence selected from SEQ ID NO:2 or SEQ ID NO:3
- a polypeptide having an amino acid sequence which differs from SEQ ID NO:2 or SEQ ID NO:3 in virtue of 1, 2, 3, 4, 5, 6 or 7 amino acid modifications, preferably in virtue of 1, 2, 3, 4, or 5 amino acid modifications and more preferably in virtue of 1, 2 or 3 amino acid modifications,

- a polypeptide which is a retro-inverso analogue of SEQ ID NO:2 or SEQ ID NO:3, namely of SEQ ID NO:7 or SEQ ID NO:8, and
- a polypeptide having an amino acid sequence which differs from SEQ ID NO:7 or SEQ ID NO:8 in virtue of 1, 2, 3, 4, 5, 6 or 7 amino acid modifications, preferably in virtue of 1, 2, 3, 4, or 5 amino acid modifications and more preferably in virtue of 1, 2 or 3 amino acid modifications. ,

The regulatory Treg peptide may comprise chemical modification in order to increase its stability and/or prevent its enzymatic degradation, in particular at its C-terminal or N-terminal end. For instance, the C-terminus of the peptide can be amidated and/or the N-terminus can be acylated.

Alternatively, the regulatory Treg peptide can be coupled to a PEG moiety

In the context of the invention, the cashew allergen is administered by epicutaneous route in combination with at least one regulatory Treg peptide selected from the group consisting of:
- a polypeptide of SEQ ID NO:2 or SEQ ID NO:3
- a polypeptide having an amino acid sequence which differs from SEQ ID NO:2 or SEQ ID NO:3 in virtue of 1, 2, 3, 4 or 5 amino acid modifications, preferably in virtue of 1, 2 or 3 amino acid modifications, and
- a polypeptide which is a retro-inverso analogue of SEQ ID NO:2 or SEQ ID NO:3, or which differs from the sequence of said retro-inverso analogue in virtue of 1, 2, 3, 4 or 5 amino acid modifications, preferably in virtue of 1, 2 or 3 amino acid modifications.

In a preferred embodiment, the cashew allergen is administered by epicutaneous route in combination with at least one regulatory Treg peptide selected from the group consisting of:
- a polypeptide of SEQ ID NO:3
- a polypeptide having an amino acid sequence which differs from SEQ ID NO:3 in virtue of 1, 2, 3, 4 or 5 amino acid modifications, preferably in virtue of 1, 2 or 3 amino acid modifications, and
- a polypeptide which is a retro-inverso analogue of SEQ ID NO:3, or which differs from the sequence of said retro-inverso analogue in virtue of 1, 2, 3, 4 or 5 amino acid modifications, preferably in virtue of 1, 2 or 3 amino acid modifications.

In a particular embodiment of the method of the invention, the cashew allergen is co-administered with at least one regulatory Treg peptide selected from a peptide of SEQ ID NO:2, a peptide of SEQ ID NO:3, a retro-inverso analogue thereof and combinations thereof.

In other embodiments, the cashew allergen is co-administered with a regulatory Treg peptide selected from a peptide of SEQ ID NO:2, a peptide of SEQ ID NO:3, and combinations thereof.

In another particular embodiment, the cashew allergen is administered by epicutaneous route in combination with a mixture of regulatory Treg peptides.

Said mixture preferably comprises (i) a polypeptide comprising or consisting of SEQ ID NO:2 or SEQ ID NO:7and (ii) a polypeptide comprising or consisting of SEQ ID NO:3 or SEQ ID NO:8.

Said mixture may further comprise one or several additional regulatory Treg peptides, in particular derived from human IgG. For instance, it may comprise one or several polypeptides comprising or consisting of a sequence selected in Table 2 of WO2008094538 or a retro-inverso version thereof. For instance, the mixture may further comprise a polypeptide comprising or consisting of SEQ ID NO:1 or its retro-inverso analogue of SEQ ID NO:6.

In some preferred embodiments, the C-terminal and N-terminal of the at least one regulatory Treg epitope are capped, preferably by amidation and acetylation accordingly.

As fully described below, the cashew allergen and the at least one regulatory Treg peptides are preferably simultaneously administered by epicutaneous, preferably in the form of an admixture optionally with one or several additional excipients, by mean of a skin patch.

### - The epicutaneous administration and means for providing such administration

Epicutaneous application is typically performed by applying the compounds of interest, namely the cashew allergen and the regulatory Treg peptide, in such a way that the compounds are in contact with the surface of the skin area in the subject. The application is maintained for a period of time sufficient to allow the penetration of the compounds of interest in the superficial layers of the skin (namely epidermis) so that they reach the epidermal dendritic cells and can be processed by said cells.

The administration of the cashew allergen and that of the regulatory Treg peptide can be performed separately, namely at a different skin area and/or at a different time. For instance, the administration of the cashew allergen and that of the regulatory Treg peptide can be performed at the same skin area or at different sites of the skin.

In a most preferred embodiment, the cashew allergen and the regulatory Treg peptide are epicutaneously delivered simultaneously, at the very same skin area in the subject.

In preferred embodiments, the compounds of interest are applied on an intact skin. More precisely the skin area does not undergo any pretreatment such as microporation, skin abrasion, stripping or chemical treatment before the application of the cashew allergen and/or the regulatory Treg peptide.

In some embodiments, the skin area is healthy, with means that skin area where the compounds of interest are applied is devoid of any skin disorder such as wound, skin irritation, skin inflammation, or skin disease such as eczema.

Epicutaneous administration is preferably performed using a device suitable to maintain contact between the compounds of interest and the skin of the subject. Such devices may include a patch, a tape, a dressing, a sheet, a gel such as hydrogel, hydrocolloids or any other form known to those skilled in the art.

The device may be suitable to deliver the compounds in the superficial layers of skin, mostly in the epidermis with no significant passage in the bloodstream.

In some embodiments, the compounds of interest may be in the form of a liquid composition and applied using known devices, such as occlusive devices having a reservoir and a perforated membrane.

In some preferred embodiments, the skin device is a patch, even more preferably an occlusive patch wherein the compounds of interest are present in dry form. For instance, the invention can be carried out by using a skin patch device as described by the Applicant in e.g., WO2011/128430; WO02/071950, or WO2007/12226.

In a specific embodiment, such a device is occlusive and is configured to use the cashew allergen and/or the regulatory Treg peptide in dry form.

In some embodiments, the patch comprises a backing wherein the cashew allergen and/or the regulatory Treg peptide is/are deposited preferably in dry form.

In some embodiments, the allergen and the regulatory Treg peptide may be maintained on the patch through electrostatic forces without the mean of any adhesive.

In a particular embodiment, the portion of the backing of the patch bearing the compounds of interest is not in direct contact with the skin. For the performance of the present invention, it is particularly suited to use a device comprising a backing adapted to create with the skin a hermetically closed condensation chamber. The compounds of interest, namely the allergen and/or the regulatory Treg peptide are deposited on this backing. The compounds of interest can be absorbed on the backing or adhered on it through electrostatic forces such as Van der Waals forces. Upon application of the patch to the skin, moisture increases in the chamber, leading to the solubilization of the allergen and the regulatory Treg peptide and contacting with the skin.

In other words, when applied on the skin, the patch forms an occlusive, impermeable chamber where the compounds of interest deposited on the inner surface of the chamber in dry form, are solubilized by trans-epidermal water loss (perspiration) and can then cross the *stratum corneum* and penetrate intro epidermis so as to reach epidermal dendritic cells. The term "perspiration" means the production of a fluid that is excreted by the sweat glands in the skin of mammals. This fluid contains mainly water but also various dissolved minerals and trace elements. In the present embodiment, perspiration secreted by the skin evaporates and condenses within the hermetically closed chamber.

It is believed that the hermetically closed chamber increases the permeability of the skin facilitating the passage of the allergen and the regulatory Treg epitope into the epidermis. Of note, the Applicant showed that such a device does not result in a significant passage of the allergens into the bloodstream.

The height of the condensation chamber defined by the backing, the periphery of the backing and the skin is in the range of 0,1 mm to 1 cm, typically from 0,1 mm to 5 mm such as 0.1 to 1 mm.

The backing (also called herein the support) of the patch may be of glass or a polymer chosen from the group consisting of cellulose plastics (CA, CP), polyvinyl chloride (PVC), polypropylenes, polystyrenes, polyurethanes, polycarbonates, polyacrylics in particular poly(methyl methacrylate (PMMA), polyesters, polyethylenes (PE), polyethylene terephthalate (PET), fluoropolymers (PTFE for example) and ethylene vinyl acrylates (EVA).

In some embodiments, the occlusive skin patch may comprise a breathable overadhesive (also called dressing), a backing preferably made of polyethylene terephthalate (PET) and an adhesive crown, the adhesive crown and the backing being adapted to create with the skin a hermetically closed chamber. The adhesive crown can be any appropriate polymeric foam recovered by an appropriate adhesive on its lower and upper faces. The foam is typically selected so as to form an airtight joint between the backing of the patch and the skin of the subject. The breathable overadhesive (or adhesive dressing) is used to ensure the adhesiveness of the patch on the skin.

The occlusive patch may further comprises a release liner and a paper applicator.

An example of an appropriate patch to carried out the invention is for example Viaskin patch developed by the Applicant and as illustrated in Figure 3.

Typically, the allergen and/or the regulatory Treg peptide are present in dry form onto the skin facing side of the backing. These compounds may have been deposed onto the backing by spray drying or by electrospray as described in WO2009095591. Alternatively, the compounds of interest can adhere to the backing by means of an adhesive e.g. an acrylic adhesive.

When electrospray is used, the backing is typically coated with a conductive layer, e.g. coated with metal particles or metal oxide particles such as titanium oxide, aluminium, or gold, so as to enable positive or negatives charges to be distributed over the backing during the electrospray process and the deposit of the compounds of interest.

The cashew allergen and the regulatory Treg peptide can be present in pure form or as an admixture with pharmaceutically acceptable excipient(s), with or without an additional adjuvant(s) in the patch. Preferably, the cashew allergen and the regulatory Treg peptide are not combined with any additional adjuvant in the patch.

In some embodiments, the cashew allergen and the regulatory Treg peptide are administered in the form of an adjuvant-free preparation by epicutaneous route.

Pharmaceutically acceptable excipients that may be used are, in particular, described in the Handbook of Pharmaceuticals Excipients, American Pharmaceutical Association (Pharmaceutical Press; 6th revised edition, 2009). Examples of appropriate excipients include, but are not limited to, solvents such as water or water/ethanol mixtures, fillers, carriers, diluents, binders, osmotic agents, salts, buffering agents, stabilizers, anti-oxidants, preservatives, surfactants, wetting agents, and the like.

For instance the compounds of interest, namely the cashew allergen and the regulatory Treg peptide may be formulated together with one or several excipients selected from buffering agents, diluents, surfactants, and stabilizers typically in the form of a liquid formulation (e.g. as an hydroalcoholic solution) said formulation being deposited, for instance by electrospray, on the skin facing backing of the patch in condition enabling the evaporation of the solvent and the formation of a dry deposit on the backing.

It goes without saying that the patch as described above is also an object of the present invention.

### - Posology and regimen

The methods of the invention comprise the repeated administration of the cashew allergen in combination with the regulatory Treg peptide to the subject by epicutaneous route, leading to a progressive increase in tolerance to cashew in the subject.

The specific dose of allergen and regulatory Treg peptide as well as the number of applications and duration of contact can be adapted by the skilled artisan, depending on the subject, the nature of the allergen/regulatory Treg peptide preparation(s), the type of device used to provide epicutaneous administration, etc.

Generally, the method comprises the application of the allergen and regulatory Treg peptide at least once a month over a period of time of several months (e.g. 6, 12, 18, 24, 36, 48 months) until a sufficient increase in tolerance is obtained.

In some embodiments, the allergen and regulatory Treg peptide are administered at least one a week, preferably once a day or every two days during a period of time of at least 6 months such as at least 1, 2 or 3 years.

In some preferred embodiments, the cashew allergen and the regulatory Treg peptide are contained within the same skin patch, preferably as described above. In that case, the method of the invention may comprise the repeated application of a patch at least once a week, e.g. every day or every two days, preferably once a day.

For instance, the method of the invention may comprise the application of a new patch comprising both the cashew allergen and the regulatory Treg peptide once every two days or every day. The duration of contact of the patch with the skin for each application is in the range of about 1 to 48 hours, typically about 3 to 36 hours, e.g., around 6 hours, 8 hours, 12 hours, 18 hours or 24 hours.

The amount of cashew allergens and regulatory Treg peptide on each patch is typically in the range of 0.1 to 1000 µg/cm² of patch surface, preferably in the range of 20 to 500 µg/cm² of patch surface, more preferably in the range of 20 to 200 µg/cm² of patch surface. The patch surface is in the range of 1 cm² to 10 cm², preferably in the range of 1 cm² to 5 cm².

The dosage of cashew allergens may be from 1 µg to 10 mg, preferably from 10 µg to 1 mg such as from 30 µg to 800 µg or 50 µg to 500 µg per patch.

The dosage of regulatory Treg peptide may be from may be from 1 µg to 10 mg, preferably from 10 µg to 1 mg such as from 30 µg to 800 µg or 50 µg to 500 µg per patch.

The weight ratio of the cashew allergen to the regulatory Treg epitope may be from 0.1 to 10, such as 0.5 to 2 or 0.8 to 1.2.

To efficiently increase the tolerance of the subject to cashew, cashew allergens and regulatory Treg peptides are administered in a dose sufficient to induce an immune reaction in the subject.

In some cases, especially in the beginning of the treatment, the application of the cashew allergen on the skin may involve a local inflammatory reaction leading to a cascade of biochemical events involving the local vascular system and the immune system. Inflammatory reaction is either moderate in the form of erythema (first clinical element of the inflammatory reaction), or in the form of a papula also indicating the presence of local edema (another component of the inflammatory reaction). The inflammatory reaction induced by the application of cashew allergens via the epicutaneous route can be visible or nonvisible to the unaided eye.

The method of invention may comprise an initiation phase wherein the contact duration of the patch with the skin is progressively increased e.g. from 3 hours a day to 12 hours a day. The initiation phase may last 1 to 4 weeks.

After the initiation phase and during the regular treatment, the patch is preferably applied once a day at least until a sufficient increase in tolerance is achieved.

Once a sufficient increase in tolerance is achieved, a maintenance treatment may be carried out. The frequency of application of the patch can be reduced in the maintenance treatment e.g. to once a week or once a month. Alternatively, the maintenance treatment may comprise the administration of the cashew allergen alone (i.e. without the regulatory Treg peptide) by epicutaneous route. The patch containing the cashew allergen alone can be applied at the same frequency as in the regular treatment (e.g. once a day) or at a lower frequency (e.g. once a week or once a month).

For application, the patch devices is applied directly to the skin, preferably on an intact area of the skin and more preferably on a dry, clean, healthy, in particular non-inflamed area of the skin. Typically the patch can be applied on shoulders, scapula, arms, or thighs.

Preferably, the skin does not undergo any pre-treatment before the application of the patch. Alternatively, the skin may be pretreated prior to application of the cashew allergens and the regulatory Treg peptide. The pretreatment of the skin is preferably contemplated when the subject is an adult. The pretreatment of the skin is preferably superficial. Typically, the skin pretreatment can alter the stratum corneum and optionally one or several epidermis cell layers while maintaining the integrity of dermis and the hypodermis of the skin. Preferably, the skin pretreatment does not alter the epidermis basal layer as well. Skin treatments encompass, without being limited to, skin microporation, such as laser microporation, skin cleansing, gentle skin stripping, skin exfoliation, and the like. When the subject is an infant or a child under the age of 12 years, skin pretreatment is avoided.

The method of the invention results in a reduction of specific IgE levels and an increase in some specific IgG levels, in particular in IgG4 levels, leading to a progressive increase in tolerance to groundnut. The term "specific Ig" refers herein to immunoglobulins which are specific to at least one allergen to which the subject is allergic. In a preferred embodiment, these immunoglobulins are specific to at least one protein derived from groundnut, especially Ana o 1, Ana o 2, Ana o 3, or their isoforms.

The method of the invention also leads to an immune deviation from a dominant Th2 profile to a more balanced Th1/Th2 profile. In other words, the method of the invention causes a raising of a Th1 response to the proteins administered. Th1 and Th2 cells are two types of CD4+ helper T-cells which differ in their pattern of cytokines production. Th1 cells produce IFN-γ, IL-2 and TNF-β and are involved in cell-mediated immune responses that are beneficial in host-defence against intracellular pathogens and malignant cells, but detrimental in mediating autoimmunity. Th2 cells secrete IL-4, IL-5, IL-9, IL-10 and IL-13, which increase antibody responses, including IgE production, and protect against parasitic infestations but can also cause allergy and asthma. Th1 and Th2 responses are mutually antagonistic, such that they normally exist in equilibrium and cross-regulate each other. In allergic subject, the balance Th1/Th2 is altered and the Th2 profile is predominant. An immune deviation from dominant Th2 profile to a more balanced Th1/Th2 profile means a deviation from an allergic state to a tolerant state. This deviation, mediated by an increase of Treg cells, can be evaluated by any method known by the skilled person, such as a decrease in ratio IgG1/IgG4 or the analysis of cytokine production.

The examples also show that the method of the invention decreases cashew-specific effector response, more precisely inhibits the proliferation of cashew-specific T cells and mast cell degranulation after oral challenge as compared to the placebo group.

The following examples are given for purposes of illustration and not by way of limitation.

### Listing of sequences

| SEQ ID | Description | Séquence |
|---|---|---|
| SEQ ID NO:1 | hTregitope from IgG1 (CH) | PAVLQSSGLYSLSSVVTVPSSSLGTQ |
| SEQ ID NO:2 | hTregitope from IgG1 (CH) | EEQYNSTYRVVSVLTVLHQDW |
| SEQ ID NO:3 | hTregitope from IgK (VL) | GTDFTLTISSLQPED |
| SEQ ID NO:4 | mTregitope from IgG1 (CH) - autologous to SEQ ID NO:1 | PAVLQSDLYTLSSSVTVPSS |
| SEQ ID NO:5 | mTregitope from IgG1 (CH) - autologous to SEQ ID NO:2 | EEQFNSTFRSVSELPIMHQ |
| SEQ ID NO:6 | Retro-inverso of hTregitope from IgG1 (CH) of SEQ ID NO:1 | QTGLSSSPVTVVSSLSYLGSSQLVAP (D-amino acid residues) |
| SEQ ID NO:7 | Retro-inverso of hTregitope from IgG1 (CH) of SEQ ID NO:2 | WDQHLVTLVSVVRYTSNYQEE (D-amino acid residues) |
| SEQ ID NO:8 | Retro-inverso of hTregitope from IgK (VL) of SEQ ID NO:3 | DEPQLSSITLTFDTG (D-amino acid residues) |

### EXAMPLES

### Combination Therapy with Tregitope and Cashew Improves & Accelerates THE Therapeutic Effects of Epicutaneous Immunotherapy in a Robust Mouse Model of Cashew Allergy

Seventy mice were sensitized by 1 mg of cashew protein extract supplemented with 10 µg of Cholera Toxin per intragastric route, at a rate of one intragastric administration per week for 6 consecutive weeks. Sensitized mice were then treated by epicutaneous immunotherapy using Viaskin patches:
- 10 mice were treated with viaskin containing excipient (Phosphate buffer 0.1 M + DMSO)
- 10 mice were treated with viaskin containing 50 µg cashew protein (Stallergenes Greer, ref XPF84D3A2.5),
- 10 mice were treated with viaskin containing 50 µg cashew protein + 50 µg T1 (comparative)
- 10 mice were treated with viaskin containing 50 µg cashew protein + 50 µg T2 (invention)
- 10 mice were treated with viaskin containing 50 µg cashew protein + 50 µg T3 (invention)
- 10 mice were treated with viaskin containing 50 µg cashew protein + 50 µg T1 + 50 µg T2+ 50 µg T3 (invention)
- 10 mice were treated with viaskin containing 50 µg T1 + 50 µg T2 + 50 µg T3 (invention).
mTregitopes T1, T2 and T3 correspond to the homologs of human Tregitopes of SEQ ID NO:1, SEQ ID NO:2 and SEQ ID NO:3, respectively. The N-terminus of the tested peptides was acetylated (CH3(C=O)-) while the C-terminus was amidated (-NH2) to prevent degradation by protease.

### - Preparation of Viaskin^{®}-excipient patches

A solution of 528 µL of Phosphate buffer 0.1 M and 72 µL of DMSOwas prepared and 50 µl volume were deposited on patches with micropipette, spread the drop on the backing layer surface of the patch, and dried in a ventilated (100%) oven at 30°C. Dried patches will be stored in a petri dishes at 5 ± 3°C with desiccant as soon as the deposition is totally dried. Viaskin^{®} were prepared by loading 50 µl of Phosphate buffer + DMSO on each patch.

Solubilization of Tregitope:
all reagents were brought to room temperature.
Vials containing Tregitope were centrifuged at 7500rpm for 1 minute.
Each peptide were diluted with 100% sterile DMSO to a working concentration of 25 mg/mL or 25 µg/µl (NET mass).
Peptides were aliquoted by 80µL in 100% DMSO and stored at -20C

Preparation of Viaskin^{®}-Cashew (prepared the day before the application):
Prepare 3 mL of a Cashew solution at 10 mg/mL and then prepare the following solutions:
Cashew + Tregitope 1: 576 µL of Cashew solution at 10 mg/mL + 24 µL of Tregitope 1 in DMSO (25mg/mL)
Cashew + Tregitope 2: 576 µL of Cashew solution at 10 mg/mL + 24 µL of Tregitope 2 in DMSO (25mg/mL)
Cashew + Tregitope 3: 576 µL of Cashew solution at 10 mg/mL + 24 µL of Tregitope 3 in DMSO (25mg/mL)
Cashew + Tregitope mix: 528 µL of Cashew solution at 10 mg/mL + 24 µL of Tregitope 1 in DMSO (25mg/mL) + 24 µL of Tregitope 2 in DMSO (25mg/mL) + 24 µL of Tregitope 3 in DMSO (25mg/mL)
Tregitope mix: 528 µL of phosphate buffer + 24 µL of Tregitope 1 in DMSO (25mg/mL) + 24 µL of Tregitope 2 in DMSO (25mg/mL) + 24 µL of Tregitope 3 in DMSO (25mg/mL)

Viaskin^{®} were prepared by loading 50 µl of corresponding solution.
the drop was spread on the backing layer surface of the patch and dried in a ventilated oven at 30°C. Dried patches were stored in a petri dishes at 5 ± 3°C with desiccant as soon as the deposition is totally dried.

Mice received VIASKIN for 48 hrs once a week for 8 weeks. 10 naïve mice were left untreated as healthy control. After 8 weeks of treatment, mice were challenged orally with 45 mg of cashew protein extract. Body temperature was measured before challenge and every 10 minutes for 60 minutes following challenge using subcutaneous transponders. Clinical symptoms were recorded following the same frequency using following score:

| **Score** | **Symptoms** | |
|---|---|---|
| **0** | No symptom | |
| **1** | Repetitive mouth/ear scratching or Ruffled fur | |
| **2** | Puffiness around eyes or Pilo-erection or Decreased activity / Prostration | |
| **3** | Dyspnea / Sneezing or Cyanosis around mouth or Diarrhea | |
| **4** | No reaction to whiskers stimulation or Convulsions | **Any animal reaching score 4 is killed** |

Anaphylactic reactions were characterized by a drop in body temperature and the occurrence of clinical symptoms. A blood sample were collected 60 min after the challenge for the measurement of mMCP-1 and mMCP-7 levels as markers of mast cell degranulation.

For antibody response analysis, blood sample of 200 µL were collected from the sub-mandibular vein at the end of the sensitization phase, after 4 weeks of treatment and at the end of experiment. Cashew-specific IgE, IgG1 and IgG2a were measured by in house ELISA.

At the end of experiment, animals were sacrificed to collect spleen and brachial lymph nodes. Cells from spleen and LN were isolated for phenotyping of Tregs by FACS (example of staining: LiveDead, CD3, CD4, CD25, FoxP3, LAP, CD62L). Cells from spleen were also used for in vitro culture with or without cashew stimulation for proliferation and cytokine production analysis.

### Results

EPIT treatment with VIASKIN containing cashew protein induced a significant decrease of mast cell degranulation after oral challenge whatever the presence of Tregitope in the VIASKIN (Figure 1A). However, patches loaded with cashew alone or cashew and T1 did not induce protection from anaphylactic reactions at this time point, i.e. 8 weeks of treatment, whereas addition of T2, T3, or mix of the 3 Tregitopes resulted in significant protection from anaphylaxis after challenge evidenced by a less pronounced drop in body temperature and lower clinical symptom scores (Figure 1B and C).

Cashew-specific IgE were significantly lower compared to control only in the group treated by combination of Cashew + T3, but IgG2a titers measured by ELISA were not different from control measurements whatever the treatment, even if a trend in higher IgG2a was observed in mice treated with cashew + T3.

Mice that had been treated with EPIT containing cashew with T2, T3, or mix of the 3 Tregitopes had increased numbers of CD62L⁺/FoxP3⁺ Tregs in skin-draining lymph nodes and in their spleens (Figure 2A and B). This increase in Tregs was associated with reduced cashew specific T-cell proliferation (Figure 2C) and a down-modulation of Th2 cytokines (IL-4) in re-stimulated splenocytes (Figure 2D).

## Claims

1. A cashew allergen for use in increasing tolerance to cashew in a subject, wherein the cashew allergen is administered in combination with at least one regulatory Treg peptide by epicutaneous route and wherein:
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof, and
- the at least one regulatory Treg peptide is able to cause a tolerogenic response and consists of:
- a polypeptide consisting of SEQ ID NO:2 or SEQ ID NO:3,
- a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
- a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications.

2. The cashew allergen for use according to claim 1 wherein the at least one regulatory Treg peptide comprises a chemical modification in order to prevent its enzymatic degradation by protease at its C-terminus and/or N-terminus, preferably amidation at C-terminus end and/or acetylation at N-terminus.

3. The cashew allergen for use according to claims 1 or 2, wherein the at least one regulatory Treg peptide is selected from the group consisting of a peptide of SEQ ID NO:2, a peptide of SEQ ID NO:3, retro-inverso analogues thereof and combinations thereof.

4. The cashew allergen for use according to any one of claims 1 to 3, wherein the at least one regulatory Treg peptide is a mixture of regulatory Treg peptides comprising:
(i) a polypeptide consisting of SEQ ID NO:2 or its retro-inverso analogue,
(ii) a polypeptide consisting of SEQ ID NO:3 or its retro-inverso analogue, and
(iii) optionally a polypeptide consisting of SEQ ID NO:1 or its retro-inverso analogue.

5. The cashew allergen for use according to any one of Claims 1 to 4, wherein the cashew allergen is a protein extract from cashew nut, preferably obtained from defatted cashew nut flour.

6. The cashew allergen for use according to any one of Claims 1 to 5, wherein the cashew allergen is for application in combination with the at least one regulatory Treg peptide by mean of a skin patch.

7. The cashew allergen for use according to Claim 6, wherein the skin patch comprises a backing having a periphery adapted to create a hermetically closed chamber when applied on the subject's skin, the cashew allergen and the at least one regulatory Treg peptide are present in dry form, optionally in admixture with one or several pharmaceutically acceptable excipients, on the backing of the patch and wherein the cashew allergen and the at least one regulatory Treg peptide are solubilized by trans-epidermal water loss whereby they can cross the *stratum corneum* and penetrate intro epidermis so as to reach epidermal dendritic cells.

8. The cashew allergen for use according to any one of Claims 1 to 7, wherein the cashew allergen is for simultaneous application with the at least one regulatory Treg epitope on a skin area of the subject, every day or every two days, by means of a skin patch, during at least 6 months, preferably at least 12 months.

9. The cashew allergen for use according to any one of claims 1 to 8, wherein the subject has been diagnosed as allergic to cashew or is at risk of being allergic to cashew and/or wherein the cashew allergen is for providing desensitization in a subject allergic to cashew.

10. A Regulatory Treg peptide for use in increasing the responsiveness of a subject to desensitization with a cashew allergen, wherein:
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof,
- the at least one regulatory Treg peptide is able to cause a tolerogenic response and consists of:
- a polypeptide consisting of SEQ ID NO:2 or SEQ ID NO:3,
- a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
- a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications, and
- the regulatory Treg peptide and the cashew allergen are administered by epicutaneous route, preferably by means of a skin patch.

11. The Regulatory Treg peptide according to claim 10 wherein the cashew allergen is as defined in claim 5 and/or the at least one regulatory Treg peptide is as defined in any one of claims 2 to 4 and/or the skin patch is as defined in claim 7.

12. A pharmaceutical combination comprising a cashew allergen and at least one regulatory Treg peptide for use in increasing cashew tolerance in a subject, wherein the combination is administered by epicutaneous route, preferably by means of a skin patch, and wherein
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof, preferably wherein the cashew allergen is as defined in Claim 5, and
- the at least one regulatory Treg peptide is able to cause a tolerogenic response and consists of:
- a polypeptide consisting of SEQ ID NO:2 or SEQ ID NO:3,
- a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
- a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications, preferably as defined in any one of claims 2 to 4.

13. A skin patch comprising a backing having a periphery adapted to create a hermetically closed chamber when applied on a subject's skin, wherein :
- the skin patch comprises a cashew allergen and at least one regulatory Treg peptide present in dry form, optionally in admixture with one or several pharmaceutically acceptable excipients, on the skin facing surface of the patch backing,
- the cashew allergen comprises at least one allergenic protein selected from the group consisting of Ana o 1, Ana o 2, Ana o 3, isoforms thereof, fragments thereof wherein the fragments comprise at least one IgE-binding epitope originally present in said proteins, and combinations thereof, and
- the at least one regulatory Treg peptide is able to cause a tolerogenic response and consists of:
- a polypeptide consisting of SEQ ID NO:2 or SEQ ID NO:3,
- a polypeptide consisting of an amino acid sequence which differs from SEQ ID NO:2 or SEQ ID NO:3 by 1, 2, 3, 4 or 5 amino acid modifications, preferably by 1, 2 or 3 amino acid modifications, or
- a polypeptide which is a retro-inverso analogue of SEQ ID NO:2 or SEQ ID NO:3, or which differs from said retro-inverso analogue by 1, 2, 3, 4 or 5 amino acid modifications, preferably by 1, 2 or 3 amino acid modifications.

14. The skin patch of Claim 13 wherein the patch comprises a backing preferably made of polyethylene terephthalate (PET) and an adhesive foam crown, the adhesive foam crown being adapted to form an airtight joint between the backing of the patch and the skin of the subject whereby a hermetically closed chamber is obtained when the patch is applied on the skin.

## Patentansprüche

1. Ein Cashew-Allergen zur Verwendung bei der Erhöhung der Toleranz gegenüber Cashew in einem Subjekt, wobei das Cashew-Allergen in Kombination mit mindestens einem regulatorischen Treg-Peptid auf epikutanem Weg verabreicht wird und wobei:
- das Cashew-Allergen mindestens ein allergenes Protein ausgewählt aus der Gruppe bestehend aus Ana o 1, Ana o 2, Ana o 3, Isofome davon, Fragmente davon umfasst , wobei die Fragmente mindestens ein urprünglich in den Proteinen vorhandes IgE-bindendes Epitop und Kombinationen davon umfassen, und
- das mindestens eine regulatorische Treg-Peptid fähig ist eine tolerogene Reaktion hervorzurufen und aus folgendem besteht:
- ein Polypeptid bestehend aus SEQ ID NO:2 oder SEQ ID NO:3,
- ein Polypeptid bestehend aus einer Aminosäuresequenz, die sich von SEQ ID NO: 2 oder SEQ ID NO: 3 durch 1, 2, 3, 4 oder 5 Aminosäuremodifikationen, bevorzugt durch 1, 2 oder 3 Aminosäuremodifikationen unterscheidet oder
- ein Polypeptid, das ein Retro-Inverso-Analog der SEQ ID NO: 2 oder SEQ ID NO: 3 ist oder das sich von dem Retro-Inverso-Analog durch 1, 2, 3, 4 oder 5 Aminosäuremodifikationen, bevorzugt durch 1, 2 oder 3 Aminosäuemodifikationen unterscheidet.

2. Das Cashew-Allergen zur Verwendung gemäß Anspruch 1, wobei das mindestens eine regulatorische Treg Peptid eine chemische Modifikation umfasst um seine enzymatische Degradation durch Protease an seinem C-Terminus und/oder N-Terminus, bevorzugt Amidierung am C-Terminus und/oder Acetylierung am N-Terminus zu verhindern.

3. Das Cashew-Allergen zur Verwendung gemäß Anspruch 1 oder 2, wobei das mindestens eine regulatorische Treg Peptid ausgewählt ist aus der Gruppe bestehend aus einem Peptid der SEQ ID NO: 2, einem Peptid der SEQ ID NO: 3, Retro-Inverso-Analogen davon und Kombinationen davon.

4. Das Cashew-Allergen zur Verwendung gemäß einem der Ansprüche 1 bis 3, wobei das mindestens eine regulatorische Treg Peptid ein Gemisch aus regulatorischen Treg Peptiden ist umfassend:
(i) ein Polypeptid bestehend aus SEQ ID NO: 2 oder sein Retro-Inverso-Analog,
(ii) ein Polypeptid bestehend aus SEQ ID NO: 3 oder sein Retro-Inverso-Analog und
(iii) optional ein Polypeptid bestehend aus SEQ ID NO: 1 oder sein Retro-Inverso-Analog.

5. Das Cashew-Allergen zur Verwendung gemäß einem der Ansprüche 1 bis 4, wobei das Cashew-Allergen ein Proteinextrakt aus Cashewnuss ist, vorzugsweise aus entfettetem Cashewnussmehl gewonnen.

6. Das Cashew-Allergen zur Verwendung gemäß einem der Ansprüche 1 bis 5, wobei das Cashew-Allergen zur Anwendung in Kombination mit mindestens einem regulatorischen Treg Peptid mittels eines Hautpflasters ist.

7. Das Cashew-Allergen zur Verwendung gemäß Anspruch 6, wobei das Hautpflaster eine Trägerfolie umfasst, deren Rand so gestaltet ist, dass beim Aufbringen auf die Haut des Subjekts eine hermetisch abgeschlossene Kammer entsteht, wobei das Cashew-Allergen und das mindestens eine regulatorische Treg-Peptid in trockener Form, optional in Mischung mit einem oder mehreren pharmazeutisch verträglichen Hilfsstoffen, auf der Trägerfolie des Pflasters vorliegen und wobei das Cashew-Allergen und das mindestens eine regulatorische Treg Peptid durch den transepidermalen Wasserverlust gelöst werden, wodurch sie die Hornschicht durchdringen und in die Epidermis eindringen können, um die dendritischen Zellen der Epidermis zu erreichen.

8. Das Cashew-Allergen zur Verwendung gemäß einem der Ansprüche 1 bis 7, wobei das Cashew-Allergen zur gleichzeitigen Applikation mit dem mindestens einen regulatorischen Treg-Epitop auf eine Hautstelle des Subjekts täglich oder alle zwei Tage mittels eines Hautpflasters über einen Zeitraum von mindestens 6 Monaten, vorzugsweise mindestens 12 Monaten, bestimmt ist.

9. Das Cashew-Allergen zur Verwendung gemäß einem der Ansprüche 1 bis 8, wobei bei dem Subjekt eine Allergie gegen Cashewnüsse diagnostiziert wurde oder es besteht das Risiko einer solchen Allergie, und/oder das Cashew-Allergen dient der Desensibilisierung einer Person, die gegen Cashewnüsse allergisch ist.

10. Ein regulatorisches Treg-Peptid zur Verwendung bei der Steigerung der Ansprechbarkeit eines Subjekts auf eine Desensibilisierung mit einem Cashew-Allergen, wobei:
- das Cashew-Allergen mindestens ein allergenes Protein umfasst, ausgewählt aus der Gruppe bestehend aus Ana o 1, Ana o 2, Ana o 3, Isoforme davon, Fragmente davon, wobei die Fragmente mindestens ein ursprünglich in den genannten Proteinen vorhandenes IgE-bindendes Epitop umfassen, sowie Kombinationen davon,
- das mindestens eine regulatorische Treg Peptid fähig ist eine tolerogene Reaktion hervorzurufen und besteht aus:
- ein Polypeptid bestehend aus SEQ ID NO: 2 oder SEQ ID NO: 3,
- ein Polypeptid bestehend aus einer Aminosäuresequenz die sich von der SEQ ID NO: 2 oder SEQ ID NO: 3 durch 1, 2, 3, 4 oder 5 Aminosäuremodifikationen, vorzugsweise durch 1, 2 oder 3 Aminosäuremodifikationen unterscheidet oder
- ein Polypeptid, das ein Retro-Inverso-Analog der SEQ ID NO: 2 oder SEQ ID NO: 3 ist oder das sich von dem Retro-Inverso-Analog durch 1, 2, 3, 4 oder 5 Aminosäuremodifikationen, bevorzugt durch 1, 2 oder 3 Aminosäuemodifikationen unterscheidet, und
- das regulatorische Treg Peptid und das Cashew-Allergen auf epikutanem Weg verabreicht werden, bevorzugt mittels eines Hautpflasters.

11. Das regulatorische Treg Peptid gemäß Anspruch 10, wobei das Cashew-Allergen ist wie in Anspruch 5 definiert und/oder das mindestens eine regulatorische Treg Peptid ist wie in einem der Ansprüche 2 bis 4 definiert und/oder das Hautpflaster ist wie in Anspruch 7 definiert.

12. Eine pharmazeutische Kombination umfassend ein Cashew-Allergen und mindestens ein regulatorisches Treg Peptid zur Verwendung bei der Erhöhung der Toleranz gegenüber Cashew in einem Subjekt, wobei die Kombination auf epikutanem Weg verabreicht wird, bevorzugt mittels eines Hautpflasters und wobei
- das Cahew-Allergen mindestens ein allergenes Protein ausgewählt aus der Gruppe bestehend aus Ana o 1, Ana o 2, Ana o 3, Isofomen davon, Fragmente davon umfasst, wobei die Fragmente mindestens ein urprünglich in den Proteinen vorhandes IgE-bindendes Epitop und Kombinationen davon umfassen, vorzugsweise wobei das Cashew-Allergen ist wie in Anspruch 5 definiert, und
- das mindestens eine regulatorische Treg Peptid fähig ist eine tolerogene Reaktion hervorzurufen und aus folgendem besteht:
- ein Polypeptide bestehend aus SEQ ID NO:2 oder SEQ ID NO:3,
- ein Polypeptid bestehend aus einer Aminosäuresequenz, die sich von SEQ ID NO: 2 oder SEQ ID NO: 3 durch 1, 2, 3, 4 oder 5 Aminosäuremodifikationen, bevorzugt durch 1, 2 oder 3 Aminosäuremodifikationen unterscheidet oder
- ein Polypeptid, das ein Retro-Inverso-Analog der SEQ ID NO: 2 oder SEQ ID NO: 3 ist oder das sich von dem Retro-Inverso-Analog durch 1, 2, 3, 4 oder 5 Aminosäuremodifikationen, bevorzugt durch 1, 2 oder 3 Aminosäuemodifikationen unterscheidet, vorzugsweise wie in einem der Ansprüche 2 bis 4 definiert.

13. Ein Hautpflaster umfassend eine Trägerfolie, deren Rand so gestaltet ist, dass beim Aufbringen auf die Haut des Subjekts eine hermetisch abgeschlossene Kammer entsteht, wobei:
- das Hautpflaster ein Cashew-Allergen und mindestens ein regulatorisches Treg-Peptid umfasst in trockener Form, optional in Mischung mit einem oder mehreren pharmazeutisch verträglichen Hilfsstoffen, auf der der Haut zugewandten Seite der Pflasterträgerfolie,
- das Cashew-Allergen mindestens ein allergenes Protein ausgewählt aus der Gruppe bestehend aus Ana o 1, Ana o2, Ana o 3, Isoforme davon, Fragmente davon umfasst, wobei die Fragmente mindestens ein urprünglich in den Proteinen vorhandes IgE-bindendes Epitop und Kombinationen davon umfassen und
- das mindestens eine regulatorische Treg Peptid fähig ist eine tolerogene Reaktion hervorzurufen und aus folgendem besteht:
- ein Polypeptid bestehend aus SEQ ID NO:2 oder SEQ ID NO:3,
- ein Polypeptid bestehend aus einer Aminosäuresequenz, die sich von SEQ ID NO: 2 oder SEQ ID NO: 3 durch 1, 2, 3, 4 oder 5 Aminosäuremodifikationen, bevorzugt durch 1, 2 oder 3 Aminosäuremodifikationen unterscheidet oder
- ein Polypeptid, das ein Retro-Inverso-Analog der SEQ ID NO: 2 oder SEQ ID NO: 3 ist oder das sich von dem Retro-Inverso-Analog durch 1, 2, 3, 4 oder 5 Aminosäuremodifikationen, bevorzugt durch 1, 2 oder 3 Aminosäuemodifikationen unterscheidet.

14. Das Hautpflaster gemäß Anspruch 13, wobei das Pflaster eine Trägerfolie, vorzugsweise aus Polyethylenterephthalat (PET), und eine Klebeschaumkrone umfasst, wobei die Klebeschaumkrone so ausgelegt ist, dass sie eine luftdichte Verbindung zwischen der Trägerfolie des Pflasters und der Haut des Subjekts herstellt, wodurch eine hermetisch abgeschlossene Kammer entsteht, wenn das Pflaster auf die Haut aufgebracht wird.

## Revendications

1. Allergène de noix de cajou pour une utilisation dans l'augmentation de la tolérance à la noix de cajou chez un sujet, lequel allergène de noix de cajou est administré en combinaison avec au moins un peptide régulateur de Treg par voie épicutanée, et dans lequel :
- l'allergène de noix de cajou comprend au moins une protéine allergénique choisie dans le groupe constitué par Ana o 1, Ana o 2, Ana o 3, leurs isoformes, leurs fragments, lesquels fragments comprennent au moins un épitope se liant aux IgE présent à l'origine dans lesdites protéines, et leurs combinaisons, et
- l'au moins un peptide régulateur de Treg est capable de provoquer une réponse tolérogénique et consiste en :
- un polypeptide consistant en la SEQ ID NO : 2 ou la SEQ ID NO : 3,
- un polypeptide consistant en une séquence d'acides aminés qui diffère de la SEQ ID NO : 2 ou de la SEQ ID NO : 3 par 1, 2, 3, 4 ou 5 modifications d'acides aminés, de préférence par 1, 2 ou 3 modifications d'acides aminés, ou
- un polypeptide qui est un analogue rétro-inverse de la SEQ ID NO : 2 ou de la SEQ ID NO : 3, ou qui diffère dudit analogue rétro-inverse par 1, 2, 3, 4 ou 5 modifications d'acides aminés, de préférence par 1, 2 ou 3 modifications d'acides aminés.

2. Allergène de noix de cajou pour une utilisation selon la revendication 1, dans lequel l'au moins un peptide régulateur de Treg comprend une modification chimique afin de prévenir sa dégradation enzymatique par une protéase au niveau de son extrémité C et/ou de son extrémité N, de préférence une amidation à l'extrémité C et/ou une acétylation à l'extrémité N.

3. Allergène de noix de cajou pour une utilisation selon la revendication 1 ou 2, dans lequel l'au moins un peptide régulateur de Treg est choisi dans le groupe constitué par un peptide de SEQ ID NO : 2, un peptide de SEQ ID NO : 3, leurs analogues rétro-inverses, et leurs combinaisons.

4. Allergène de noix de cajou pour une utilisation selon l'une quelconque des revendications 1 à 3, dans lequel l'au moins un peptide régulateur de Treg est un mélange de peptides régulateurs de Treg comprenant :
(i) le polypeptide consistant en la SEQ ID NO : 2 ou son analogue rétro-inverse,
(ii) un polypeptide consistant en la SEQ ID NO : 3 ou son analogue rétro-inverse, et
(iii) éventuellement un polypeptide consistant en la SEQ ID NO : 1 ou son analogue rétro-inverse.

5. Allergène de noix de cajou pour une utilisation selon l'une quelconque des revendications 1 à 4, lequel allergène de noix de cajou est un extrait protéique provenant de la noix de cajou, de préférence obtenu à partir de farine de noix de cajou dégraissée.

6. Allergène de noix de cajou pour une utilisation selon l'une quelconque des revendications 1 à 5, lequel allergène de noix de cajou est destiné à être appliqué en combinaison avec l'au moins un peptide régulateur de Treg au moyen d'un patch cutané.

7. Allergène de noix de cajou pour une utilisation selon la revendication 6, dans lequel le patch cutané comprend un envers ayant une périphérie adaptée pour créer une chambre hermétiquement fermée quand il est appliqué sur la peau du sujet, l'allergène de noix de cajou et l'au moins un peptide régulateur de Treg sont présents sous forme sèche, éventuellement en mélange avec un ou plusieurs excipients pharmaceutiquement acceptables, sur l'envers du patchet dans lequel l'allergène de noix de cajou et l'au moins un peptide régulateur de Treg sont solubilisés par l'eau trans-épidermique, ce par quoi ils peuvent traverser le *stratum corneum* et pénétrer dans l'épiderme de façon à atteindre les cellules dendritiques épidermiques.

8. Allergène de noix de cajou pour une utilisation selon l'une quelconque des revendications 1 à 7, lequel allergène de noix de cajou est destiné à être appliqué simultanément avec l'au moins un épitope régulateur de Treg sur une zone de peau du sujet, chaque jour ou tous les deux jours, au moyen d'un patch cutané, pendant au moins 6 mois, de préférence au moins 12 mois.

9. Allergène de noix de cajou pour une utilisation selon l'une quelconque des revendications 1 à 8, dans lequel le sujet a été diagnostiqué comme étant allergique aux noix de cajou ou risquant d'être allergique à la noix de cajou, et/ou lequel allergène de noix de cajou est pour fournir une désensibilisation chez un sujet allergique à la noix de cajou.

10. Peptide régulateur de Treg pour une utilisation dans l'augmentation de la réponse d'un sujet à une désensibilisation avec un allergène de noix de cajou, dans lequel :
- l'allergène de noix de cajou comprend au moins une protéine allergénique choisie dans le groupe constitué par Ana o 1, Ana o 2, Ana o 3, leurs isoformes, leurs fragments, lesquels fragments comprennent au moins un épitope se liant aux IgE présent à l'origine dans lesdites protéines, et leurs combinaisons,
- l'au moins un peptide régulateur de Treg est capable de provoquer une réponse tolérogénique et consiste en :
- un polypeptide consistant en la SEQ ID NO : 2 ou la SEQ ID NO : 3,
- un polypeptide consistant en une séquence d'acides aminés qui diffère de la SEQ ID NO : 2 ou de la SEQ ID NO : 3 par 1, 2, 3, 4 ou 5 modifications d'acides aminés, de préférence par 1, 2 ou 3 modifications d'acides aminés, ou
- un polypeptide qui est un analogue rétro-inverse de la SEQ ID NO : 2 ou de la SEQ ID NO : 3, ou qui diffère dudit analogue rétro-inverse par 1, 2, 3, 4 ou 5 modifications d'acides aminés, de préférence par 1, 2 ou 3 modifications d'acides aminés, et
- le peptide régulateur de Treg et l'allergène de noix de cajou sont administrés par voie épicutanée, de préférence au moyen d'un patch cutané.

11. Peptide régulateur de Treg selon la revendication 10, dans lequel l'allergène de noix de cajou est tel que défini dans la revendication 5 et/ou l'au moins un peptide régulateur de Treg est tel que défini dans l'une quelconque des revendications 2 à 4 et/ou le patch cutané est tel que défini dans la revendication 7.

12. Combinaison pharmaceutique comprenant un allergène de noix de cajou et au moins un peptide régulateur de Treg pour une utilisation dans l'augmentation de la tolérance à la noix de cajou chez un sujet, laquelle combinaison est administrée par voie épicutanée, de préférence au moyen d'un patch cutané, et dans laquelle
- l'allergène de noix de cajou comprend au moins une protéine allergénique choisie dans le groupe constitué par Ana o 1, Ana o 2, Ana o 3, leurs isoformes, leurs fragments, lesquels fragments comprennent au moins un épitope se liant aux IgE présent à l'origine dans lesdites protéines, et leurs combinaisons, de préférence lequel allergène de noix de cajou est tel que défini dans la revendication 5, et
- l'au moins un peptide régulateur de Treg est capable de provoquer une réponse tolérogénique et consiste en :
- un polypeptide consistant en la SEQ ID NO : 2 ou la SEQ ID NO : 3,
- un polypeptide consistant en une séquence d'acides aminés qui diffère de la SEQ ID NO : 2 ou de la SEQ ID NO : 3 par 1, 2, 3, 4 ou 5 modifications d'acides aminés, de préférence par 1, 2 ou 3 modifications d'acides aminés, ou
- un polypeptide qui est un analogue rétro-inverse de la SEQ ID NO : 2 ou de la SEQ ID NO : 3, ou qui diffère dudit analogue rétro-inverse par 1, 2, 3, 4 ou 5 modifications d'acides aminés, de préférence par 1, 2 ou 3 modifications d'acides aminés,
de préférence tel que défini dans l'une quelconque des revendications 2 à 4.

13. Patch cutané comprenant un envers ayant une périphérie adaptée pour créer une chambre hermétiquement fermée quand il est appliqué sur la peau d'un sujet, dans lequel :
- le patch cutané comprend un allergène de noix de cajou et au moins un peptide régulateur de Treg présents sous forme sèche, éventuellement en mélange avec un ou plusieurs excipients pharmaceutiquement acceptables, sur la surface faisant face à la peau de l'envers du patch,
- l'allergène de noix de cajou comprend au moins une protéine allergénique choisie dans le groupe constitué par Ana o 1, Ana o 2, Ana o 3, leurs isoformes, leurs fragments, lesquels fragments comprennent au moins un épitope se liant aux IgE présent à l'origine dans lesdites protéines, et leurs combinaisons, et
- l'au moins un peptide régulateur de Treg est capable de provoquer une réponse tolérogénique et consiste en :
- un polypeptide consistant en la SEQ ID NO : 2 ou la SEQ ID NO : 3,
- un polypeptide consistant en une séquence d'acides aminés qui diffère de la SEQ ID NO : 2 ou de la SEQ ID NO : 3 par 1, 2, 3, 4 ou 5 modifications d'acides aminés, de préférence par 1, 2 ou 3 modifications d'acides aminés, ou
- un polypeptide qui est un analogue rétro-inverse de la SEQ ID NO : 2 ou de la SEQ ID NO : 3, ou qui diffère dudit analogue rétro-inverse par 1, 2, 3, 4 ou 5 modifications d'acides aminés, de préférence par 1, 2 ou 3 modifications d'acides aminés.

14. Patch cutané selon la revendication 13, lequel patch comprend un envers de préférence fait en poly(téréphtalate d'éthylène) (PET) et une couronne de mousse adhésive, la couronne de mousse adhésive étant adaptée pour former un joint étanche à l'air entre l'envers du patch et la peau du sujet, ce par quoi une chambre hermétiquement fermée est obtenue quand le patch est appliqué sur la peau.
